# EUROPEAN PATENT SPECIFICATION

(11) **EP 2 994 739 B1**
(45) Date of publication and mention of the grant of the patent: **19.10.2016**
(21) Application number: 14729596.8
(22) Date of filing: 28.04.2014
(51) Int. Cl.: G01N 11/00, G01N 33/32

(54) **METHOD FOR DEMONSTRATING THE NON-DRIP PROPERTIES OF A HAIR DYE**
VERFAHREN ZUM DEMONSTRIEREN DER TROPFFESTIGKEITEIGENSCHAFTEN EINES HAARFÄRBEMITTELS
PROCÉDÉ POUR DÉMONTRER LES PROPRIÉTÉS ANTI-GOUTTE D'UN COLORANT CAPILLAIRE

(30) Priority: 09.05.2013 EP 13167200
(43) Date of publication of application: 16.03.2016
(73) Proprietor: Unilever PLC, London, Greater London EC4Y 0DY (GB); Unilever N.V., 3013 AL Rotterdam (NL)
(72) Inventor: DAWSON, Robert, Wayne, Wirral Merseyside CH63 3JW (GB); DESALE, Shirish, Subhash, Wirral Merseyside CH63 3JW (GB); WYRKO, Andrew, David, Wirral Merseyside CH63 3JW (GB)
(74) Representative: James, Helen Sarah
(86) International application number: PCT/EP2014/058630
(87) International publication number: WO 2014/180699

(56) References cited:
- EP-A1- 0 894 491
- JP-A- 2005 247 742
- SU-A1- 913 159

## Description

The present invention relates to a method for demonstrating the non-drip aspects of a hair colouring formulation. If a hair dye does not drip, it is easy to apply and is less likely to stain clothes and skin.

EP 0 894 491 discloses a method for demonstrating the non-drip properties of a hair dye composition, whereby the hair dye to be tested is applied to the hair of test persons.

JP 2005 247742 discloses a method for demonstrating the non-drip properties of a hair dye composition, whereby the hair dye to be tested is applied to the hair of wigs.

There remains a need for methods to demonstrate the non-drip properties of hair colouring compositions under well-defined and repeatable conditions.

Accordingly the present invention relates to a method for demonstrating the non-drip properties of a hair dye composition comprising the following steps in this order:
i) application of a hair dye composition to a stencil, the stencil being placed over a smooth horizontal substrate;
ii) tilting the smooth substrate to an angle from 20 to 60 degrees from a horizontal plane;
iii) removal of the stencil from the smooth surface;
iv) visualization of movement of the hair dye composition on the substrate.

The tilting of the substrate causes compositions that have a greater tendency to drip, to run down the substrate and thus distort the original image left by the stencil.

The smooth surface is tilted at angle elevation from 20 to 60 degrees, more preferably from elevation degrees from 25 to 30 degrees. In the context of the present application elevation angle is defined as the angle of the tilt of the smooth surface relative to a horizontal plane.

Preferably, the substrate is a non-absorbent material; more preferably the substrate is formed from glass or acryl, more preferably acryl.

It is preferable if the substrate is a dark colour or black, more preferably it is black.

Preferably, the stencil is a non-absorbent material; more preferably the stencil is formed from glass or acryl, more preferably acryl. Preferably the stencil is transparent.

Preferably the shape of the cut out of the stencil is that of several letters forming a word. Preferably the stencil has more than one word cut out, this enables a comparison of products. More preferably the number of cut outs relating to words is from 2 to 5, most preferably the cut outs represents 3 words.

Preferably the stencil has a depth (depth of cut out) from 2mm to 8mm, more preferably from 4mm to 6mm.

Preferably the hair dye composition is placed in the cut out of the stencil using a spatula or pipette. Preferably the hair dye composition is placed such that the complete area of the cut out of the stencil is covered.

Preferably the method can demonstrate the ease of spreading of more than one hair dye composition.

Preferably the composition to be used with a method is a mixture of a first composition and a second composition. The first composition comprising an oxidative dye, and the second composition comprising a per-oxygen generating material. When the two mixtures are mixed at a weight ratio from 1:3 to 3:1, more preferably at a weight ratio from 1:2 to 2:1 most preferably at a weight ratio of 3:2 to 2:3 the resulting mix has:
a) a yield stress from 75 to 150Pa, preferably from 85 to 125 and more preferably from 90 to 110 Pa;
b) a viscosity from 0.1 to 0.5 PaS, preferably from 0.15 to 0.4 and more preferably from 0.2 to 0.35;
c) preferably a thixotropic recovery from 60 to 100%, more preferably from 70 to 80%;
d) preferably a thixotropic breakdown from 20 to 190 seconds (S), more preferably from 30 to 180 (S).

### EXAMPLE 1

A clear acryl stencil, having 3 cut outs relating to product names, is placed over horizontal back acryl substrate. 3 hair dyes are placed such that they totally fill the cutouts using a spatula or pipettes as appropriate. Each composition is placed in cutout of a word representing the compositions name. The stencil and substrate are tilted to a 28 degree angle relative to a horizontal plane. The stencil is removed from the substrate. Initially the compositions names are clearly seen, however the resulting movement of the image on the substrate demonstrates tendency of the composition to drip. Thus the composition which is least likely to drip renders a clearly seen name for longer as the composition does not run down the board.

## Claims

1. A method for demonstrating the non-drip properties of a hair dye composition comprising the following steps in this order:
i) application of a hair dye composition to a stencil, the stencil being placed over a smooth horizontal substrate;
ii) tilting the smooth substrate to an angle elevation from 20 to 60 degrees in relation to a horizontal plane;
iii) removal of the stencil from the smooth substrate;
iv) visualization of movement of the hair dye composition on the substrate.

2. Method according to claim 1 wherein the smooth substrate is acrylic .

3. Method according to any preceding claim in which the smooth substrate is black.

4. Method according to any preceding claim in which the smooth substrate is tilted to an angle from 25 to 30 degrees in relation to a horizontal pane.

5. Method according to any preceding claim in which the stencil has cut outs representing words.

6. Method according to any preceding claim in which the stencil is acrylic.

7. Method according to any preceding claim in which the stencil is transparent.

8. Method according to any preceding claim where the hair dye is placed into the cut out of the stencil with a spatula or pipette.

9. Method according to any preceding which simultaneously demonstrates the non-drip properties of more than one hair dye composition.

10. Method according to claim 7 in which the stencil has more than one word representing different product names.

11. Method according to any preceding claim in which at least one hair dye composition has a has a yield stress from 75 to 150Pa; a viscosity from 0.1 to 0.5 PaS.

## Patentansprüche

1. Verfahren zum Nachweis der Tropffestigkeitseigenschaften einer Haarfärbezusammensetzung, umfassend die folgenden Schritte in dieser Reihenfolge:
i) Aufbringen einer Haarfärbezusammensetzung auf eine Schablone, wobei die Schablone über einem glatten horizontalen Substrat angeordnet ist,
ii) Neigen des glatten Substrats auf einen Winkelanstieg von 20 bis 60 Grad in Bezug auf eine horizontale Ebene,
iii) Entfernen der Schablone von dem glatten Substrat,
iv) Visualisieren der Bewegung der Haarfärbezusammensetzung auf dem Substrat.

2. Verfahren nach Anspruch 1, wobei das glatte Substrat Acryl ist.

3. Verfahren nach irgendeinem vorhergehenden Anspruch, in welchem das glatte Substrat schwarz ist.

4. Verfahren nach irgendeinem vorhergehenden Anspruch, in welchem das glatte Substrat in einem Winkel von 25 bis 30 Grad in Bezug auf eine horizontale Ebene geneigt wird.

5. Verfahren nach irgendeinem vorhergehenden Anspruch, in welchem die Schablone Wörter darstellende Aussparungen aufweist.

6. Verfahren nach irgendeinem vorhergehenden Anspruch, worin die Schablone Acryl ist.

7. Verfahren nach irgendeinem vorhergehenden Anspruch, in welchem die Schablone transparent ist.

8. Verfahren nach irgendeinem vorhergehenden Anspruch, wobei das Haarfärbemittel mit einem Spatel oder einer Pipette in die Aussparung der Schablone eingebracht wird.

9. Verfahren nach irgendeinem vorhergehenden Anspruch, welches gleichzeitig die Tropffestigkeitseigenschaften von mehr als einer Haarfärbezusammensetzung nachweist.

10. Verfahren nach Anspruch 7, in welchem die Schablone mehr als ein Wort aufweist, was verschiedene Produktnamen darstellt.

11. Verfahren nach irgendeinem vorhergehenden Anspruch, in welchem mindestens eine Haarfärbezusammensetzung eine Fließspannung von 75 bis 150 Pa und eine Viskosität von 0,1 bis 0,5 Pas aufweist.

## Revendications

1. Procédé pour démontrer les propriétés anti-goutte d'une composition de teinture pour cheveux comprenant, les étapes suivantes, dans cet ordre, consistant à :
i) appliquer une composition de teinture pour cheveux sur un pinceau, le pinceau étant placé sur un substrat horizontal lisse ;
ii) incliner le substrat lisse à un angle de 20 à 60 degrés par rapport à un plan horizontal ;
iii) retirer le pinceau du substrat lisse ;
iiv) visualiser le mouvement de la composition de teinture pour cheveux sur le substrat.

2. Procédé selon la revendication 1, dans lequel le substrat lisse est en acrylique.

3. Procédé selon l'une quelconque des revendications précédentes, dans lequel le substrat lisse est noir.

4. Procédé selon l'une quelconque des revendications précédentes, dans lequel le substrat lisse est incliné à un angle de 25 à 30 degrés par rapport à un plan horizontal.

5. Procédé selon l'une quelconque des revendications précédentes, dans lequel le pinceau a des découpes représentant des mots.

6. Procédé selon l'une quelconque des revendications précédentes, dans lequel le pinceau est en acrylique.

7. Procédé selon l'une quelconque des revendications précédentes, dans lequel le pinceau est transparent.

8. Procédé selon l'une quelconque des revendications précédentes, dans lequel la teinture pour cheveux est placée sur la découpe du pinceau avec une spatule ou une pipette.

9. Procédé selon l'une quelconque des revendications précédentes, qui démontre simultanément les propriétés anti-goutte de plus d'une composition de teinture pour cheveux.

10. Procédé selon la revendication 7, dans lequel le pinceau a plus d'un mot représentant différents noms de produit.

11. Procédé selon l'une quelconque des revendications précédentes, dans lequel au moins une composition de teinture pour cheveux a une limite d'écoulement de 75 à 150 Pa ; une viscosité de 0,1 à 0,5 PaS.
